# EUROPEAN PATENT APPLICATION

(11) **EP 1 731 177 A1**
(43) Date of publication of application: **13.12.2006**
(21) Application number: 05719186.8
(22) Date of filing: 16.02.2005
(51) Int. Cl.: A61L 27/00, C12N 5/06

(54) **BIOLOGICAL TISSUE SHEET, METHOD OF FORMING THE SAME AND TRANSPLANTATION METHOD BY USING THE SHEET**

(30) Priority: 11.03.2004 JP 2004069097
(71) Applicant: ArBlast Co., Ltd., Kobe Hyogo 650-0047 (JP); Hashimoto, Kouji, Touon-shi Ehime 791-0314 (JP)
(72) Inventor: HASHIMOTO, Kouji, Touon-shi, Ehime 791-0314 (JP); SHIRAKATA, Yuuji, Touon-shi, Ehime 791-0204 (JP); OHASHI, Yuuichi Crystal Court Dougo 92-307, Matsuyama-shi, Ehime 790-0833 (JP); HAMURO, Junji, Yokohama-shi Kanagawa 241-0813 (JP)
(74) Representative: Müller Fottner Steinecke
(86) International application number: PCT/JP2005/002334
(87) International publication number: WO 2005/087286

(57) **Abstract**

A biological tissue sheet which is expected as exerting a favorable therapeutic effect and a high safety in transplantation. The biological tissue sheet formed by (a) preparing in vivo-derived cells; (b) sowing the in vivo-derived cells on amniotic membrane; and (c) culturing and proliferating the in vivo-derived cells in the absence of any xenogeneic animal cells. As the cells of a biological origin, for example, cells originating in corneal epithelium, conjunctival epithelium, skin epidermis, hair follicle epithelium, oral mucosa, respiratory tract mucosa, or intestinal tract mucosa.

## Description

### TECHNICAL FIELD

The present invention relates to a biological tissue sheet. More specifically, the present invention relates to a biological tissue sheet produced by culturing and proliferating in vivo-derived cells such as corneal and conjunctival epithelial cells, epidermal cells, hair follicle epithelial cells, oral mucosa epithelial cells, respiratory tract mucosa epithelial cells, intestinal tract mucosa epithelial cells, and the like, on amniotic membrane without using cells derived from xenogeneic animals as feeder cells, a method of forming the sheet and use (a transplantation method, and the like) of the sheet.

### BACKGROUND ART

The skin is an organ that covers the outermost layer of organism and forms a kind of barrier for protecting organism from the outside. The skin is made up of epidermis, dermis and subcutaneous tissue. The epidermis mainly consists of keratinocytes and includes a small amount of pigment cells, langerhans cells, and the like. Cells constituting the epidermis are mainly keratinocytes and include (1) cornified cells from which nucleus has disappeared, which occupy the outermost layer, and (2) cells (granular cells, spinous cells, and basal cell) having nucleus, which are located under the cornified cells. Further, epidermal basal membrane exists between the basal cells of the bottom layer and dermis. The basal layer is a monolayer, which includes a layer of mother cells of the epidermal keratinocytes. It is thought that a cell capable of being divided is located only in the basal cell layer.

A condition in which epidermis is defective by some reasons is ulcer. In a region in which epidermis has been lost, epidermis is regenerated by proliferation of keratinocytes from the vicinity or proliferation of keratinocytes derived from partial hair follicle, and the ulcer surface becomes epithelium. However, in a burn injury in which a wide range of epidermis is lost at one time or in highly refractory ulcer from which hair follicle is lost, it takes a long time to generate epithelium only by regeneration of the epidermis from the vicinity.

The cell cycle of the cell located in the basal cell layer is about 450 hours. Daughter cells generated by division change in terms of form and function when they move to spinous cell layers. Furthermore, they change to granule cell layers, are then cornified to become horny cell layers, and finally dropped off to the outside of the body. The time until cells are dropped off is referred to as a turnover time, which is thought to be 47 to 48 days.

In a regeneration model of epidermis, which is currently recognized in general, the epidermal keratinocyte is classified into three kinds of cells: a stem cell, a transit-amplifying cell, and a post-mitotic cell in accordance with the division ability. The stem cell has infinite self-generative ability and generates a transit-amplifying cell by division. The transit-amplifying cell has a certain division ability and becomes a transit-amplifying cell after division, but it finally loses the division ability and becomes a post-mitotic cell.

The stem cell of the epidermis is thought to have the following features: (1) the stem cell itself has a long cell cycle (slow cycling); (2) the stem cell exists in different place depending upon the regions; (3) the stem cell assembles; and (4) the stem cell strongly expresses α2β1 and α3β1 integrin. However, about 40% of the basal cells strongly express these integrins and the stem cells are actually estimated to be about 10% of the basal cells. It means that the basal layer includes not only stem cells but also transit - amplifying cells and post - mitotic cells.

When the epidermis is damaged, proliferative stimulus occurs, so that cells start to proliferate actively and regeneration of cells starts. At this time, a series of epidermal growth factors play the most important role. The epidermal growth factor (EGF) family includes EGF, transforming growth factor (TGF) - α, heparin binding EGF like growth factor (HB - EGF), betacellulin, amphiregulin, and neuregulins. The factors actually playing a role in the proliferation of the epidermal keratinocytes are thought to be TGF-α, HB-EGF, and amphiregluin. These factors are shown to act on self-proliferation of the epidermal keratinocytes. On the contrary, as the factors acting in suppressing of proliferation of the epidermal keratinocytes, TGF-β, vitamin D₃, retinoic acid, and the like are known.

In the field of regenerative medicine, grafts to be transplanted to a damaged site, in which full thickness of dermis and epithelium in human skin is lost, have been under development. For example, Japanese Patent Unexamined Publication No. H10-277143 (patent document 1) describes a graft for treating wound in which full thickness of the human skin is lost, and the like, and a method of manufacturing the graft. The graft disclosed in the document is formed by embedding fibroblasts derived from the dermis tissue into a human fibrin sheet and attaching the epidermal tissue on the surface of the sheet.
Conventionally, repairing of skin loss includes full layer skin graft transplantation in which full thickness skin is transplanted and split layer skin graft transplantation in which the epidermis and the upper layer of the dermis are transplanted. However, there is a limitation to the size of collected cite. In this point, it is advantageous that in the case of cultured epidermis, several times larger area of skin can be obtained only by collecting a small piece of skin. Furthermore, since the cultured skin can be cryopreserved, it can be used repeatedly, which is thought to be the most advantageous point as compared with conventional transplantation methods.
The epidermal sheet is classified into an autologous cultured epidermal sheet and a xeno or allogeneic cultured epidermal sheet. The autologous cultured epidermal sheet transplantation often has a main object to cover the epidermis defective site with the autologous cultured epidermal sheet and allowed the sheet to survive. On the other hand, xeno or allogeneic cultured epidermal sheet transplantation often expects the effect as a biological dressing material. The basic study of the epidermal cell has clarified that the epidermal keratinocyte produces various cell growth factors and cytokines. The effectiveness of the xeno or allogeneic cultured epidermal sheet has been clarified.

If technologies for supplying cultured epidermis safely and stably are established, regenerative medicine together with the progress of transplantation technologies has come to be practically used, which would bring about significant benefit to patients. When the regeneration of the epidermis is taken into consideration, it is extremely reasonable to separate and culture the basal cells having dividing and proliferation ability, proliferate a large amount of cells, and regenerate the epidermis for use in transplantation. However, since the basal cells include not only a stem cell but also transit - amplifying cells, post - mitotic cells, in order to form the cultured skin efficiently, establishment of technology for selectively culturing the epidermal stem cells has been desired.
To date, many researchers have attempted to study and develop technologies for culturing epidermal cells and the results thereof have brought about much benefit in the field of skin biology. Specifically, by using cultured keratinocytes, properties of epidermal cells, regeneration, differentiation and proliferation mechanism of the epidermis has becoming clarified. On the other hand, a small piece of skin from a patient is collected, and keratinocytes can be subcultured so as to proliferate a large amount of cells. Furthermore, since the cultured cells can be cryopreserved, the cultured skin has been used for the treatment of a large range of burns, refractory ulcers, and the like.

As well as the skin, the cornea is one of the tissues to which regenerative medicine is expected to contribute. The cornea is located in the outermost layer of the optical system constituting the eyeball and is a transparent tissue having no blood vessels. The cornea contributes to obtaining a good visual acuity by forming a smooth surface along with tear. Furthermore, keratoconjunctival epithelial cell is usually brought into contact with the outside and has an effect of protecting the eyeball from foreign objects such as microorganism in the outside, ray such as ultraviolet ray, and the like. That is to say, the keratoconjunctival epithelial cells play an extremely important role of protecting the transparency of the corneal and the entire eyeball so as to maintain homeostasis.

The cornea may become not transparent by conditions such as keratitis, cornea ulcer, punch, and the like, and the transparency may be lost. With respect to the permanent deterioration of visual acuity due to the loss of transparency of cornea, treatment of transplanting a cornea that has been supplied from a donor of the eyeball is carried out. The transplantation of the cornea is carried out by transplanting the transparent cornea after removing the patient's cornea whose transparency has been lost. This transplantation recovers the transparency and enables the visual acuity to be recovered again.
Although such cornea transplantation offers an effective treatment effect, there are some diseases that cannot be treated only by transplantation of the cornea. An example of such diseases includes Stevens-Johnson syndrome, ocular pemphigoid, chemical injury, burn, and the like. In general, the keratoconjunctival epithelial cell divides every day, and old cells are peeled off and new cells are regenerated from the stem cell tissue. However, it has come to be reported that in the above-mentioned conditions, the stem cell tissue for regenerating the cornea has become impaired.

The stem cell tissue for regenerating the corneal epithelium is referred to as "corneal limbus tissue" and localized in the boundary portion between black and white eye and in the specific environment exposed to the outside. In the above-mentioned pathologic conditions, it is thought that this stem cell tissue itself undergoes some impairment and become deficient. Then, due to this deficiency of the stem cell tissue, the defective portion is covered with the conjunctiva epithelium existing around the defective portion. Thus, the transparency is lost, resulting in extreme deterioration of the visual acuity. In this way, in the above-mentioned pathologic conditions, since the corneal limbus is deleted, even if only the cornea is transplanted, the transplanted cornea cannot be maintained for a long term. Therefore, in order to reconstruct the ocular surface permanently, it is necessary that the corneal limbus is also transplanted. As one of the methods of transplanting this corneal limbus, a method of transplanting amniotic membrane has been developed (see Medical Asahi, September, 1999: p62-65, N Engl J Med 340: 1697~1703, 1999: non patent document 1). Amniotic membrane to be used for this transplantation can be obtained from the placenta of, for example, a pregnant woman who underwent caesarean section. Since amniotic membrane has thick basal membrane, in transplantation, it acts as a substrate on which the keratoconjunctival epithelial cells proliferate and differentiate. Amniotic membrane hardly has immunogenicity. In addition, amniotic membrane has effects such as anti-inflammation and suppression of cicatrisation. The keratoconjunctival epithelium and the stem cells thereof, which are transplanted on the amniotic membrane, are free from rejection of a transplantation recipient.

[Patent document 1] Japanese Patent Unexamined Publication No. H10-277143
[Non-patent document 1] Medical Asahi, September, 1999: p62-65, N Engl J Med 340: 1697 to 1703. 1999

### DISCLOSURE OF INVENTION

### [Problems to be Solved by the Invention]

Culturing of the epidermal keratinocyte has been thought to be difficult and it had not been carried out until Rheinwald and Green reported a mouse 3T3 feeder layer method in 1975. This method was not easily carried out because this method employed the mouse 3T3 cells as a feeder layer, and because use of fetal bovine serum caused difference in lots. In 1980s, Hennings and Yuspa showed that lowering Ca²⁺ concentration in the culture medium made the epidermal keratinocyte be in an undifferentiated state so as to enhance the proliferation. Furthermore, Boyce and Ham developed a MCDB 153 medium that is a low Ca²+ medium, showing that the addition of bovine pituitary gland extract enables serum free culture of human epidermal keratinocytes. In 1986, Pittelkow et al. cultured epidermal keratinocyte by using a serum free culture method, followed by changing the medium to a fetal bovine serum-added high calcium medium so as to produce a cultured epidermal sheet. When the cultured epidermal sheet is transplanted to patient with burn, an excellent performance was obtained. At present, the method of Rheinwald and Green is mainly employed. However, this method uses 3T3 cells originating in xenogeneic animals as a feeder layer. In a graft obtained by culturing cells in coexistence of cells derived from xenogeneic animals, a product of xenogeneic animal origin may exist. Therefore, transplantation using this is regarded as xenogeneic transplantation or the same and is regarded to have a big problem from the viewpoint of ethics and safety. In fact, in the medical field, xenogeneic transplantation has never been put into practical use.

The cultured epidermal sheet transplantation can cover a wide range of wound surface by sub-culturing keratinocytes from the skin having the size of a stamp. Furthermore, since the cultured cells can be cryopreserved, they can be applied for treatment of refractory and recurrent ulcer, which needs transplantation repeatedly. It is not necessarily easy to allow a cultured sheet produced by a conventional culture method, for example, transplanted cultured epidermal sheet to survive well. This is because the cultured epidermal sheet does not have a constitutional component of the basal membrane and the stratified epidermis does not form a strong horny cell layer. In this point, three-dimensional cultured skin developed by Bell is found to have a horny cell layer and a granule cell layer and is the nearest to human skin at the present time. However, this skin requires complicated technique and specific technology. This skin has not been prevailed in this country. This three-dimensional cultured skin has shown to be effective in allogeneic transplantation and has been merchandised in overseas countries. However, in the allogeneic transplantation, the effect of promoting epithelization is observed but the permanent survival of the graft cannot be expected. In Japan, in the respect of confirming the ethical and safe points, there is little prospective that hetero transplantation becomes popular, so that focus has been put on the improvement of auto transplantation.

On the other hand, as surgical treatment for ocular surface diseases in which the cornea is covered with conjunctival epithelium and becomes opacified, at present, cornea epithelium transplantation is carried out. However, in refractory keratoconjunctival diseases with strong inflammation (Stevens-Johnson syndrome, ocular pemphigoid, corneal erosion, and the like), the prognosis is extremely bad. The prime reason is that allogenic (allo) cornea epithelium having strong antigenicity is recognized and rejected by an immune system of a host. Furthermore, a complication caused by systemic or local application of a large amount of immunosuppressant agent after operation for prevention of rejection reaction is also a large factor of unfavorable prognosis. On the other hand, use of allo cornea epithelium has a problem of shortage of the number of the donors. When the technology capable of producing several tens of cornea sheets by using cornea obtained from one eye is realized, the problem of shortage of the number of the donors can be solved.

### [Means to Solve the Problems]

In view of the above-mentioned circumstances and problems, the present invention was made. The objective of the present invention is to provide a biological tissue sheet by which high therapeutic effect can be expected and which offers high degree of safety when transplantation is carried out. In order to achieve such a objective, the present inventors firstly have attempted to produce a cultured epidermal sheet. Specifically, in view of safety, under the conditions where cells (feeder cells) derived from xenogeneic animals are not used when epithelial cells are cultured, as a developed system of a conventional cultured epidermal sheet auto-transplantation, three-dimensional cultured skin was produced and then immunohistological and electron-microscopic investigation was carried out with respect to a basal membrane constituting component, cell adhesive molecule, and differentiated antigen. As a result, as compared with a conventional cultured epidermal sheet, the formation of strong horny cell layer is found, and the cell adhesion molecule and differentiation marker are sufficiently expressed similar to vivo epidermis. As to the basal membrane component, hemidesmosomes were well formed and pemphigoid antigen and β4 integrin were sufficiently expressed.
The survival of the cultured epidermal sheet is affected by the formation of the component of the basal membrane. That is to say, the cultured epidermal sheet is cultured in a state in which it is brought into close contact with the bottom surface of a plastic petri dish and it is necessary that the cultured epidermal sheet is peeled off from the bottom surface of the petri dish when the sheet is produced. This peeling is carried out by using dispase or collagenase and these enzymes decomposes the component of the basal membrane. According to reports to date, due to dispase, pemphigoid antigen cannot be detected by western blotting, and in fluorescent antibody technique, laminin 5 recognized by GB3 monoclonal antibody is degraded. Furthermore, it is reported that collagenase reduces the expression of anchoring fiber or IV type collagen. VII type collagen, but dose not affect α6β4 integrin. On the contrary, a three-dimensional cultured skin keeps a structure which is similar to in vivo epidermis and the formation of component of a basal membrane is confirmed. The investigation by the present inventor demonstrated that β1 integrin, β4 integrin and pemphigoid antigen are formed relatively favorably. Also from the findings of electron microscopy, it was confirmed that hemidesmosome is formed favorably.

Next, the present inventors have investigated whether or not transplantation materials applicable for other tissues can be produced by the same technique as for the cultured epidermal sheet. Specifically, the present inventors have attempted to produce a corneal epithelial sheet. As a result, by culturing corneal epithelial cells on amniotic membrane placed on collagen containing fibroblasts, favorable stratification and epithelization have been achieved without using a feeder cell.
As mentioned above, the present inventors have succeeded in producing safe and practical biological tissue sheet without using cells derived from xenogeneic animals at all. Furthermore, the present inventors have found that a biological tissue sheet can be produced even under the conditions of serum free culture.

Furthermore, the present inventors carried out various experiments with the assumption that a particularly excellent culture substrate for epidermal cells is amniotic membrane. Firstly, amniotic membrane is brought into close contact with collagen gel containing fibroblasts and human epidermal keratinocyte is plated thereon. Then, the present inventors observed the migration of human epidermal keratinocyte to the surrounding. As a result, the present inventors found that the migration was significantly proceeded as compared with the case (control group) where cells were directly plated on a collagen gel containing fibroblasts. On the other hand, human epidermal keratinocytes cultured on the amniotic membrane placed on the collagen gel containing fibroblasts were collected together with the amniotic membrane, followed by placing it on another amniotic membrane that is brought into close contact with a collagen gel containing fibroblasts. Then, the migration of human epidermal keratinocytes to the surrounding was observed. As a result, the migration was significantly proceeded as compared with the case (control group) where cells were directly plated on a collagen gel containing fibroblasts. These experimental results shows that on the amniotic membrane, proliferation of epidermal keratinocytes is excellent and migration ability of cells is also well exhibited. That is to say, it was clarified that amniotic membrane was extremely excellent as a culture substrate for the epidermal keratinocyte. Furthermore, based on the findings, it is thought that the following two transplantation techniques (1) and (2) are shown to be an excellent reconstruction method. (1) A method of transplanting a sheet construct (the sheet construct includes amniotic membrane (first amniotic membrane) and another amniotic membrane (second amniotic membrane) that is attached to one surface of the first amniotic membrane and further includes a cell layer partly covering the first amniotic membrane and partly covering the second amniotic membrane) to the epidermis defective portion. The sheet construct is obtained by collecting epidermal keratinocytes cultured on amniotic membrane placed on collagen gel containing fibroblasts together with the amniotic membrane, followed by placing them on another amniotic membrane and culturing thereof. (2) A method of collecting epidermal keratinocytes cultured on amniotic membrane placed on a collagen gel containing fibroblasts together with the amniotic membrane and transplanting them on another amniotic membrane which has been transplanted on skin defective portion in advance. Herein, in the case of loss of the full thickness dermis and loss including subcutaneous tissue, conservative treatment could not make epithelium. Therefore, it is necessary to firstly prepare a matrix for transplantation. For loss of dermis, artificial dermis has been conventionally used and treatment effect have been obtained to some extent. In the case of the artificial dermis, regeneration of blood vessels are observed. However, when cultured epidermis is transplanted on the artificial dermis, it is pointed out that survival is not favorable because of insufficient configuration of the basal membrane. On the other hand, when the above-mentioned transplantation technique (1) or (2) is used in combination with the transplantation of artificial dermis, high survival can be expected because cultured epidermis is transplanted via amniotic membrane having components of the basal membrane. Moreover, because a state in which the cultured epidermis is formed on amniotic membrane is obtained, after transplantation, excellent proliferation and migration to the surrounding of cells forming a cultured epidermis are promoted. As a result, cultured epidermis is extended at high speed. Thus, high treatment effect can be obtained. Therefore, even with respect to skin loss in a large range of subcutaneous tissue, by combining artificial dermis transplantation and the above-mentioned transplantation technique, it is expected that treatment, which is also excellent from the cosmetic viewpoint, can be established.

The present invention was made based on the above-mentioned results and findings and provides the following configurations.
That is to say, the present invention provides a biological tissue sheet including in vivo-derived cells proliferated on amniotic membrane in the absence of a xenogeneic animal cell.
In one embodiment of the present invention, in vivo-derived cells are proliferated in a state in which the amniotic membrane is placed on a collagen gel containing human fibroblasts. Thus, the improvement in the proliferation rate of the in vivo-derived cells is to be achieved.
As a medium for proliferating in vivo-derived cells, (1) a serum free medium, or (2) a medium including only serum derived from a recipient as a serum component may be used.
The in vivo-derived cells is preferably cells derived from corneal epithelium, conjunctival epithelium, skin epidermis, hair follicle epithelium, oral mucosa, respiratory tract mucosa or intestinal tract mucosa.
It is preferable that amniotic membrane from which epithelium has been removed is used for the amniotic membrane as a culture substrate.

In one embodiment of the present invention, the biological tissue sheet includes amniotic membrane as a culture substrate in addition to the proliferated cells.
The biological tissue sheet of the present invention may be transplanted to a tissue deficient portion, for example, directly or via amniotic membrane that is different from the amniotic membrane used as a culture substrate. In the latter case, typically, with respect to the tissue defective portion, amniotic membrane (second amniotic membrane, that is, amniotic membrane that is different from the amniotic membrane used for forming a biological tissue sheet) is transplanted, followed by transplanting the biological tissue sheet on the amniotic membrane.
A biological tissue sheet in a further embodiment of the present invention includes cells obtained by placing in vivo-derived cells, which are proliferated on amniotic membrane placed on a collagen gel containing human fibroblasts, onto a second amniotic membrane together with the amniotic membrane in the absence a heterogeneous animal cell, and further by proliferating thereof.

The present invention further provides a method for forming a biological tissue sheet. One embodiment of the forming method of the present invention includes the following steps: (a) preparing in vivo-derived cells; (b) sowing the in vivo-derived cells on amniotic membrane; and (c) culturing and proliferating the in vivo-derived cells in the absence of a xenogeneic animal cell.
In one embodiment of the present invention, as the step (b), the following step is carried out: (b-1) culturing human fibroblasts in a collagen gel; and (b-2) placing amniotic membrane on the collagen gel, followed by plating the in vivo-derived cells onto the amniotic membrane.
In another embodiment of the present invention, (d) after the in vivo-derived cells are proliferated, bringing the outermost surface layer into contact with air is carried out. With this step, keratinization (epithelization) of the cell layer is promoted.
Another embodiment of the present invention further includes (e) collecting the in vivo-derived cells together with the amniotic membrane; and (f) placing the collected in vivo-derived cells and the amniotic membrane on a second amniotic membrane with the side of the amniotic membrane facing downward, followed by culturing and proliferating the in vivo-derived cells. That is to say, two-stage culture is carried out.
As the medium used for carrying out the step (c), (1) a serum free medium, or (2) a medium including only serum derived from a recipient as a serum component may be used.
The in vivo-derived cells are preferably cells derived from corneal epithelium, conjunctival epithelium, skin epidermis, hair follicle epithelium, oral mucosa, respiratory tract mucosa or intestinal tract mucosa.
It is preferable that amniotic membrane from which epithelium has been removed is used for amniotic membrane as a culture substrate.

The present invention further provides a method of preparing a skin epidermal cell, which includes the following steps: (A) plating skin epidermal cells onto amniotic membrane; (B) culturing and proliferating the skin epidermal cells; and (C) collecting the proliferated epidermal cells.

### BRIEF DESCRIPTION OF THE DRAWINGS

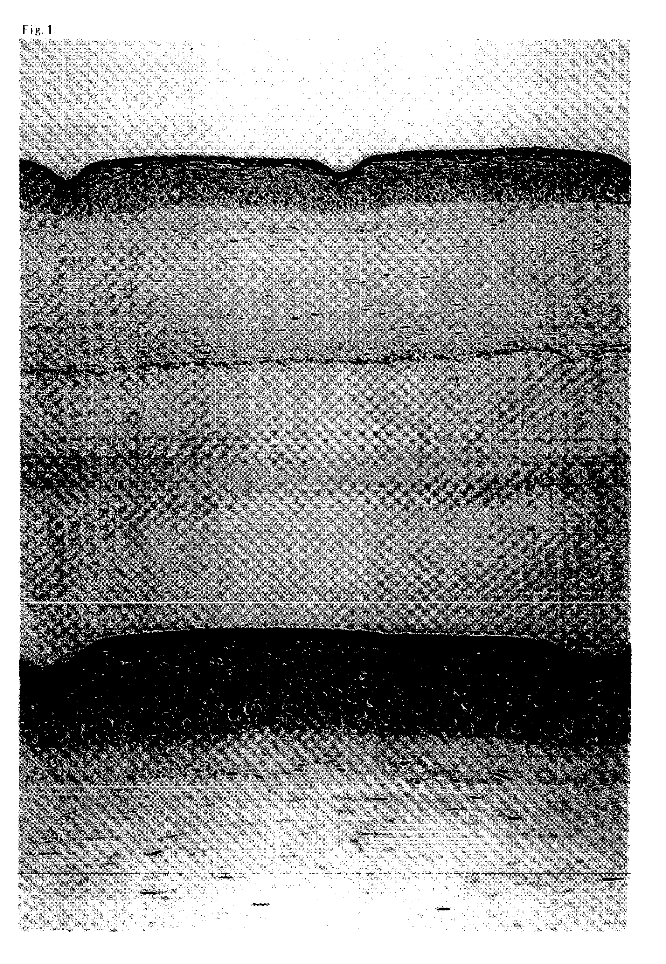

Fig. 1 shows a hematoxylin- and eosin- stained image (on day 8 following air lifting) of a three-dimensional cultured skin sheet (cultured epidermal sheet) formed by a method described in Example 1. An epithelial keratinocyte layer, amniotic membrane, and collagen gel (matrix) containing fibroblasts are shown sequentially from the upper layer. In the epidermal keratinocyte layer, a basal cell layer, a spinous cell layer, a granule cell layer, and a horny cell layer are formed in good order sequentially from the bottom, which shows a form which is very similar to that of the normal human epidermis.

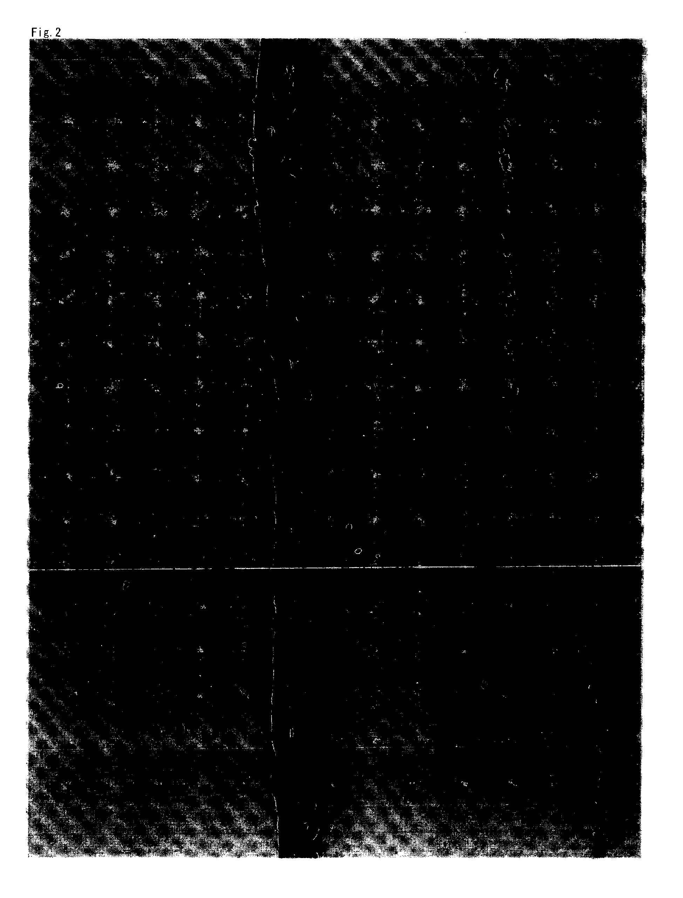

Fig. 2 shows a hematoxylin- and eosin- stained image (on day 8 following air lifting) of a three-dimensional cultured skin sheet (cultured epidermal sheet) formed by the method of Example 2. The human epidermal keratinocyte plated on the amniotic membrane includes one basal cell layer and 5 to 6 cell layers. The formation of a horny cell layer is observed and epidermis is reconstructed.

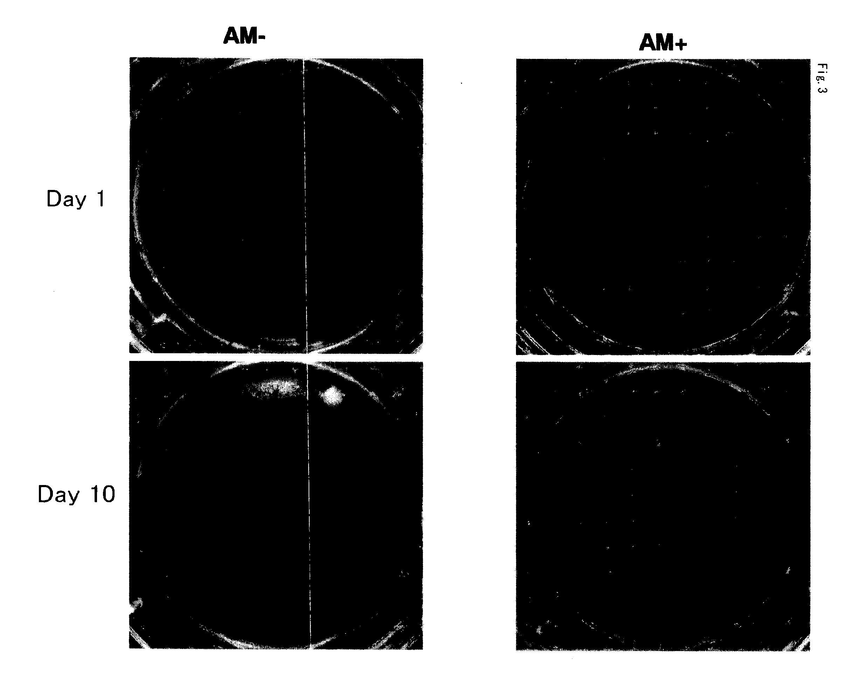

Fig. 3 shows test results of effects of amniotic membrane on proliferation and migration of epidermal keratinocytes. Right pictures show the results of the test group (a case where epidermal keratinocyte is cultured on amniotic membrane attached to collagen gel containing fibroblasts) (the upper pictures show the results on Day 1 and the lower pictures the results on Day 10). Left pictures show the results of control groups.

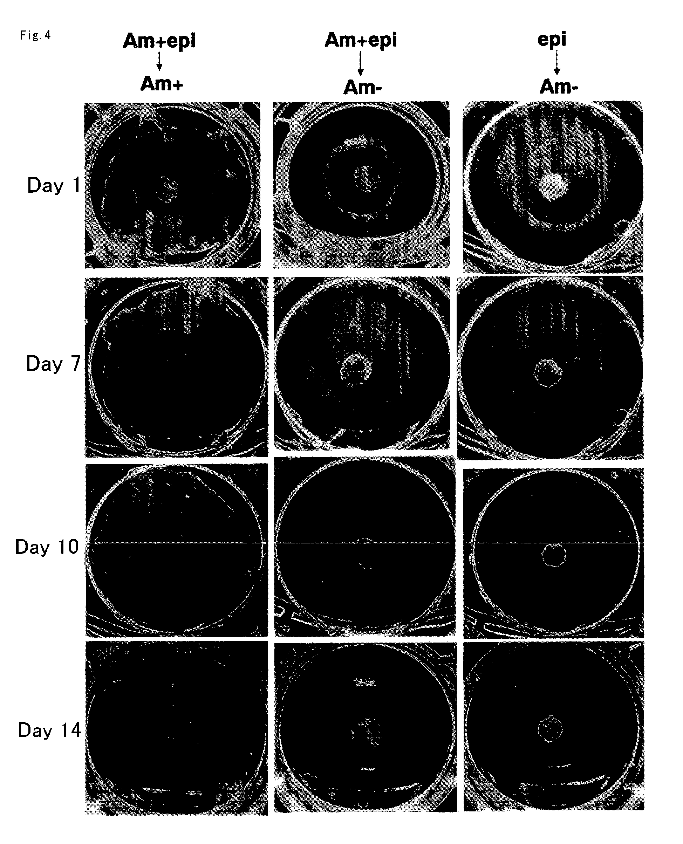

Fig. 4 shows test results of effects of amniotic membrane on proliferation and migration of epidermal keratinocyte. Left pictures show the results of the test group (a case where a cultured epidermal sheet is placed and cultured on amniotic membrane attached to collagen gel containing fibroblasts) (pictures on day 1, day 7, day 10, and day 14 are shown sequentially from the upper picture). Middle pictures show the results of a control group 1 (a case where a cultured epidermal sheet is placed and cultured on collagen gel containing fibroblasts). Similarly, right pictures show the result of a control group 2 (a case where a cell layer obtained by three-dimensionally culturing without using amniotic membrane is placed and cultured on collagen gel containing fibroblasts).

### BEST MODE FOR CARRYING OUT THE INVENTION

The present invention provides a biological tissue sheet having the following structure. That is to say, it provides a biological tissue sheet comprising in vivo-derived cells proliferated on amniotic membrane in the absence of a xenogeneic animal cell. The in vivo-derived cells form a cell layer. Hereinafter, the biological tissue sheet of the present invention and a forming method thereof are described in detail.

The biological tissue sheet of the present invention is produced by a method including (a) preparing in vivo-derived cells; (b) plating the in vivo-derived cells on amniotic membrane; and (c) culturing and proliferating the in vivo-derived cells in the absence of a xenogeneic animal cell.
In the step (a), in vivo-derived cells are prepared. As the in vivo-derived cells, cells applicable to the use of finally obtained biological tissue sheet are used. For example, when a sheet for regeneration of the skin epidermal tissue, epidermal cells (including a stem cell and a precursor cell thereof) and hair follicle epithelial cells (including a stem cell and a precursor cell thereof) are preferably used. Similarly, for the purpose of regenerating a cornea epithelial tissue, corneal epithelial cells (including a stem cell and a precursor cell thereof) are used, and for the purpose of regenerating a mucosal epithelial tissue, mucosa epithelial cells (including a stem cell and a precursor cell thereof) are preferably used. An example of the mucosa epithelial cell includes an oral mucosa epithelial cell, an intestinal tract mucosa epithelial cell, a respiratory tract mucosa epithelial cell, and the like.
A method for preparing in vivo-derived cells are described taken a skin epidermal cell, a corneal epithelial cell, an oral mucosa epithelial cell, an intestinal tract mucosa epithelial cell, and a respiratory tract mucosa epithelial cell as examples.

### (Skin Epidermal Cell)

Firstly, when the skin is collected, a site to be collected is disinfected with disinfectant such as povidone iodine prophylactically in advance and antifungal agent is externally applied thereto, followed by collecting a small skin piece in accordance with skin biopsy. In culturing epidermal keratinocytes, fatty tissue and dermis are removed from the skin piece as much as possible by using scissors and washed with Dulbecco's phosphate buffer (PBS) several times and soaked in 70% ethanol for one minute for sterilization. The piece is cut in a strip shape, soaked in Dispase solution and stood still over night at 4°C. Then, epidermis is peeled off from dermis. The peeled epidermis is washed, followed by disentangling the epidermal piece so as to prepare suspending solution of epidermal keratinocyte. The cells are suspended in a serum free medium and plated on a collagen-coated petri dish. Thus, subculture is carried out.

### (Corneal epithelial cell)

Corneal epithelial cells can be obtained from a corneal limbus tissue. For example, endothelial cells are peeled off and removed from corneal limbus tissue, and conjunctiva is excised so as to form a single cell suspension. Then, this is preserved in a nitrogen tank, and then rapidly melted at 37°C so as to adjust a corneal epithelial cell suspending solution. If necessary, subculture is carried out. For subculture, for example, EpiLife™ (Cascade Biologics), an MCDB153 medium (NISSUI PHARMACEUTICAL CO., LTD.), which are serum free media, and medium produced by modifying the amino acid composition, etc. of these media can be used.

### (Oral mucosa epithelial cell)

As the oral mucosal epithelial cells, cells existing in the dental root part (oral crevicular mucosal epithelial cells), cells of labial part, cells of palate part, cells of buccal part, and the like, can be used. Among them, it is particularly preferable to use oral crevicular mucosal epithelial cells because of the high proliferation ability and low antigenicity. The oral mucosal epithelial cells can be collected by ablating a site where targeted cells exist with the use of a scalpel, or by scraping it out. Oral crevicular mucosal epithelial cells can be collected by separating oral mucosal epithelial cells from the enamel cement transition portion and collecting the cells from the obtained tissue piece. Note here that in order to remove impurities such as connective tissue, preferably, a treatment with enzyme such as dispase or trypsin, etc., filtration treatment are carried out.

### (Intestinal tract mucosa epithelial cell)

The intestinal tract mucosa epithelial cells are collected from intestinal tract epithelium tissue through an endoscope of the large intestine, or by usual technique at the time of abdominal section. Furthermore, epithelial cells can be sectioned from tissue by laser capture microdissection. The technique of the present invention can be applied to a biological tissue sheet produced by using all the human digestive tract epithelial cells such as esophagus, upper stomach, duodenum, small intestine, and large intestine. When ulcer, inflammation, or the like, causes injuries of human digestive tract epithelium, cells derived from bone marrow play a roll as a rescue with respect to emergency, so that the epithelium is repaired. The digestive tract epithelial cells, although part of them, are also made of bone marrow. In this sense, the present invention is regarded to have significance equivalent to that using corneal epithelial cells. In general, an epithelial cell made of bone marrow, which is usually only several cells of 1000, are increased 50 to 100 times in the process in which ulcer (wound) generated by, for example, gastric ulcer, colitis, on the internal surface of the digestive tract. It is determined that about 1 of 10 digestive tract epithelial cells are derived from the bone marrow. The biological tissue sheet derived from the digestive tract mucosa epithelial cells are extremely significant because they urge the regeneration of intestinal tract epithelium with respect to ulcer and inflammation of intestine diseases which are designated intractable diseases, that is, severe intestinal tract infectious diseases such as ulcerous colitis, Crohn's disease, Behchet's disease, and the like. The effectiveness with respect to intestinal tract allergy can be expected.

### (Respiratory tract mucosa epithelial cell)

Respiratory tract mucosa epithelial cells can be obtained from biopsy tissue of the respiratory tract mucosa. Similar to the above-mentioned tissue, for removing impurities such as connective tissue, it is preferable that treatment with enzyme such as Dispase, trypsin, and the like, or filter treatment is curried out. The respiratory tract mucosa epithelial cells play an important role for pathologic conditions of various infectious diseases via biosyntheses and release of β defensin. Furthermore, respiratory tract mucosal epithelium also plays an important role in asthma or allergic disease. Providing biological tissue sheet produced by the respiratory tract mucosa epithelial cells of the present invention to the respiratory tract mucosa having tissue disorder would lead to providing artificial respiratory tract beyond to emergency treatment. In particular, immunosuppression effect of the sheet produced on the amniotic membrane is useful.

It is preferable that after tissue is collected, oral mucosa epithelial cells, intestinal tract mucosa epithelial cells, and the like, are subjected to treatment with enzyme such as Dispase, trypsin, and the like, or filter treatment in order to remove impurities such as connective tissue.

It is preferable that the in vivo-derived cells are prepared from a person (recipient) who undergoes transplantation. That is to say, it is preferable that a donor of the in vivo-derived cells are identical to a recipient of the biological transplantation sheet. By using such autologous cells, unfavorable immunological rejection is avoided.

The prepared in vivo-derived cells are seeded on amniotic membrane (the step b) and then subjected to culture (the step c).
"Amniotic membrane" is a membrane covering the outermost layer of the uterus and the placenta in mammals, and a basal membrane and an epithelium layer are formed on parenchymal tissue that is rich in collagen. It is preferable that human amniotic membrane is used as amniotic membrane. Human amniotic membrane can be collected by, for example, human embryonic membrane, placenta, etc. obtained at the time of afterbirth at delivery. Specifically, the human amniotic membrane can be prepared by treating and purifying the integrated material including human embryonic membrane, placenta, and umbilical cord obtained right after delivery. For treating and purifying, well-known method, for example, a method described in Japanese Patent Unexamined Publication No. H5-5698, etc. can be employed That is to say, amniotic membrane is detached from the embryonic membrane obtained at delivery and remaining tissue is removed by a physical treatment such as ultrasonic cleansing and an enzyme treatment, and the like. Then, appropriate cleaning process is carried out and thus the human amniotic membrane can be prepared.
The thus prepared human amniotic membrane can be cryopreserved before use. The human amniotic membrane can be frozen in a liquid mixing equal volume ratio of DMEM (Dulbecco's modified Eagle's medium) and glycerol at, for example, -80°C. By the cryopreservation, not only the improvement in operation but also reduction in the antigenicity can be expected.

Intact amniotic membrane may be used but it is preferable that amniotic membrane from which epithelium has been removed by a scraping treatment, etc. is used. For example, cryopreserved human amniotic membrane is thawed and then subjected to a treatment with EDTA or proteolytic enzyme so as to loosen the adhesion between cells. Then, epithelium is scraped by using a cell scraper, etc. Thus, the human amniotic membrane from which epithelium has been removed can be prepared.
When the amniotic membrane from which epithelium has been removed is used, it is preferable that the in vivo-derived cells are seeded on the side of the surface from which epithelium has been removed and which is exposed (that is, the side of the basal membrane). It is thought that this side of the surface is rich in type IV collagen and the in vivo-derived cells seeded are proliferated and stratified well.

The in vivo-derived cells can be plated on the amniotic membrane so that, for example, the cell density becomes about 1×10³ cells/cm² or more, preferably in the range from about 1×10³ cells/cm² to about 1×10⁷ cells/cm², and further preferably in the range from about 1×10⁴ cells/cm² to about 1×10⁶ cells/cm².

In one preferable embodiment, the amniotic membrane is placed on a collagen matrix containing human fibroblasts, which has been previously prepared, and then the in vivo-derived cells are seeded on the amniotic membrane and cultured. That is to say, in this embodiment, a step of culturing the human fibroblasts in a collagen gel (the step b-1) and a step of placing the amniotic membrane on the collagen gel, followed by sowing (placing) the in vivo-derived cells on the amniotic membrane (the step b-2) are carried out. The biological tissue sheet that has been produced by this procedure has come to contain the in vivo-derived cells proliferated on the amniotic membrane placed on the collagen gel containing human fibroblasts. The biological tissue sheet of this embodiment can be also used as a transplantation material including a collagen matrix. Furthermore, after the collagen matrix is removed, it can be used as the transplantation material. Alternatively, after the collagen matrix and the amniotic membrane are removed, it can be used as the transplantation material.
"Collagen gel" functions as a culture substrate of human fibroblasts. The kinds of collagens as a material of the collagen gel are not particularly limited, and type I collagen, type III collagen, and type IV collagen, and the like, can be used. A plurality of collagens can be used in combination thereof. Such collagens can be extracted and purified from the connective tissue of the skin and cartilage, etc. of animals such as swine, bovine, sheep, etc., by an acid solubilization method, alkali solubilization method, and oxygen solubilization method, and the like. Note here that for the purpose of deteriorating the antigenicity, it is preferable that a so-called atherocollagen obtained by removing telopeptide by a treatment with the use of catabolic enzyme such as pepsin, trypsin, etc. As materials of the collagen gel, a collagen derived from amniotic membrane, particularly derived from human amniotic membrane may be used. Herein, the collagen layer is "derived from amniotic membrane" broadly means that the collagen gel is obtained by using amniotic membrane as a starting material.

The origin of the human fibroblasts contained in the collagen gel is not particularly limited and it may be derived from any tissue as long as the tissue produces collagen. Human fibroblasts prepared from, for example, skin tissue, oral mucosal tissue, and the like, can be used.

A specific example of the method of producing a collagen matrix is shown. Firstly, human fibroblasts are prepared by the following procedure. The skin is collected, and then dermis is peeled off from the skin. The dermis is cut in strips and is brought into close contact with a dish coated with type I collagen. After static culture, human fibroblasts migrated from the dermis strip are subcultured. The cells are peeled off from the bottom surface of the dish so as to prepare a cell suspension, which is plated on a cell culture dish. Appropriately, cells are cryopreserved (for example, stored in liquid nitrogen).
On the other hand, a neutralized collagen solution is prepared using type I collagen (see the below-mentioned Example). This is added in a culture container (for example, a culture insert) and stood still for ten minutes at room temperature so as to be gelled. Next, human fibroblasts in a logarithmic growth phase, which has been cultured by the above-mentioned method in advance, is mixed with this gel and gelled again. Thereafter, static culture is carried out. A collagen matrix containing human fibroblasts can be obtained by the above-mentioned procedure. This inventiveness allows the collagen matrix to have necessary strength and to have amniotic membrane layer or in vivo-derived cells to be mounted thereon, which forms a base of the present invention. A separately prepared amniotic membrane can be placed on (brought into contact with) the collagen matrix.

Culturing of the in vivo-derived cells seeded on the amniotic membrane (the step c) is carried out in the absence of a xenogeneic animal cell. In the present invention, "in the absence of a heterogeneous animal cell" means that cells heterogeneous to the in vivo-derived cells are not used when culturing the in vivo-derived cells. Specifically, the conditions include that when human cells (for example, human epidermal cells or human corneal epithelial cells) are used as the in vivo-derived cells, cells from non-human animals such as mouse, rat, and the like, do not exist (coexist). By culturing under such a condition, finally obtained transplantation materials (that is, biological tissue sheet) may not contain components of xenogeneic species origin (including heterogeneous cells themselves).
The culture medium used for culturing in vivo-derived cells is not particularly limited as long as it allows the cells to proliferate. An example of such a medium includes MCDB 153 medium (NISSUI PHARMACEUTICAL CO., LTD.), EpiLife™ (Cascade Biologics), a medium prepared by modifying the amino acid composition, etc. of these media, and a medium, in which DMEM (Dulbecco's modified Eagle's medium) that is generally used for growing epithelial cells and Ham's F12 medium are mixed with each other at the predetermined ratio, and the like. In particular, in the present invention, it is preferable to use a medium that does not contain serum and protein of xenogeneic animal origin. On the other hand, a medium to which a growth factor, antibiotics, and the like, are added may be used. However, a medium that does not contain serum is preferable. That is to say, in a culturing method of the present invention, it is preferable to employ a serum free culture method. This is advantageous because a problem such as immunological rejection caused by contamination of components derived from serum can be avoided. Note here that culturing may be carried out in a medium containing serum, however in this case, it is preferable to use serum of the same species origin (when human in vivo-derived cells are cultured, serum of human origin is used) or to use autologous serum. Of course, if possible, it is preferable to use autologous serum that may not cause immunological rejection.
For the purpose of proliferating in vivo-derived cells well, culturing conditions can be changed in the middle of the culture step.

As a result of the step c, in vivo-derived cells proliferate on the amniotic membrane. When the surface layer of the thus obtained cell layer is required to be keratinized (for example, a case where epidermal cells are used so as to form a skin epithelial sheet or a case where corneal epithelial cells are used so as to from a corneal epithelial sheet), a step of bringing the surface layer of the cell layer into contact with air (the step d) is carried out.
Note here that this step is referred to as air-lifting in this specification. This step d is carried out in order to differentiate the cells forming a cell layer and induce a barrier function.
This step can be carried out by temporarily removing a part of a culture solution by using a dropping syringe, a pipette, and the like, so as to lower the surface of the culture solution, and thus temporarily exposing the outermost layer of the cell layer to the outside of the culture solution. Alternatively, this step can be carried out by lifting the cell layer included in amniotic membrane so as to temporarily expose the outermost layer from the surface of the culture solution. Furthermore, by sending the air into the culture solution by using a tube, etc., the outermost layer of the cell layer may be brought into contact with the air. From the viewpoint of ease in operation, it is preferable that the outermost layer of the cell layer is exposed by lowering the surface of the culture solution.
The duration of carrying out this step d, that is, the time for which the outermost layer of the cell layer is brought into contact with the air changes depending upon the state of cells and culture conditions. However, for example, it is about three days to three weeks, preferably, five days to two weeks, and more preferably about one week.

In one embodiment of the present invention, after the step c (culturing step), further steps (e) and (f) are carried out: (e) collecting the in vivo-derived cells together with the amniotic membrane; and (f) placing the collected in vivo-derived cells and the amniotic membrane on second amniotic membrane with a side of the amniotic membrane facing downward, followed by culturing and proliferating the in vivo-derived cells. By carrying out the steps e and f, it is possible to obtain a construct in which another amniotic membrane (second amniotic membrane) is attached to one amniotic membrane (first amniotic membrane), and a part of the cell layer covers the first amniotic membrane and the other part of the cell layer covers the second amniotic membrane. Note here that the size of the second amniotic membrane and culture conditions and the like are appropriately adjusted so that either a construct in which the entire surface of the second amniotic membrane is covered with a cell layer or a construct in which the surface of the second amniotic membrane is partially covered with a cell layer can be obtained.

In the step e, in vivo-derived cells cultured and the amniotic membrane used as a culture substrate are collected. For example, when in vivo-derived cells are cultured on the amniotic membrane placed in the culture dish, by peeling the amniotic membrane from the culture dish after culturing, proliferated in vivo-derived cells and the amniotic membrane can be collected. On the other hand, when the in vivo-derived cells are cultured on the amniotic membrane placed on the collagen gel, by peeling the amniotic membrane from the collagen gel after culturing, the proliferated in vivo-derived cells and the amniotic membrane can be collected.

In the step f, the collected in vivo-derived cells and the amniotic membrane are placed on another amniotic membrane and then the in vivo-derived cells are cultured again. Thus, in the embodiment, two-stage culturing (the step c and the step f) is carried out.
In the step f, firstly, a construct including the collected in vivo-derived cells and the amniotic membrane (hereinafter, referred to as "cell-amniotic membrane construct") is placed on the amniotic membrane (second amniotic membrane) with the side of the amniotic membrane facing downward. A plurality of cell-amniotic membrane constructs can be placed on the second amniotic membrane. For example, by cutting the cell-amniotic membrane construct collected after culturing in the step c, a plurality of cell-amniotic membrane constructs can be obtained. Alternatively, by preparing a plurality of culture systems that are independent from each other, and carrying out the step c with respect to the culture systems in parallel, a plurality of cell-amniotic membrane constructs can be obtained.
When a plurality of cell-amniotic membrane constructs are placed on the second amniotic membrane, it is preferable that cell-amniotic membrane constructs are disposed in a state in which they are uniformly diffusing, that is, at a predetermined interval. In the later culturing (and after transplantation to the living body), when cells are proliferated and a cell layer expands to the surrounding, in the second amniotic membrane, a region in which a cell layer was not formed at the first time can be covered quickly and efficiently. That is to say, by employing the above-mentioned method, on the second amniotic membrane, wide area of cell layer can be formed for a short time. Thus, more efficient production of cell layers becomes possible. On the other hand, before a cell layer covering the entire surface of the second amniotic membrane is formed, the cell layer including second amniotic membrane can be transplanted to a tissue deficient site of the cell layer. In this case, after transplantation, quick formation of cell layers can be promoted and high treatment effect can be obtained.
Since a wide area of cell layer can be obtained for a short time, the above-mentioned method is suitable for producing a cultured epithelial sheet. Note here that when it is necessary that the surface layer of the cell layer is keratinized, before the step e, between the step e and the step f, or after the step f, air lifting (the step d) is carried out by the same method as mentioned above.

The culturing in the step f can be carried out under the same conditions as in the above-mentioned step c. That is to say, it is preferable that culturing is carried out in the absence of a xenogeneic animal cell. It is preferable to use a medium, which is free of serum and does not contain protein derived from xenogeneic animals. When a medium containing serum is used, it is preferable to use serum of the same species origin (when cells of human biological origin are cultured, serum of human origin is used) or to use autologous serum. Furthermore, similar to the step c, also in the step f, for the purpose of well proliferating the in vivo-derived cells, the culturing conditions may be changed in the middle of the culturing step.

The biological tissue sheet of the present invention is extremely safe because xenogeneic animal cells are not used in the production process and the amniotic membrane is used as a scaffold when in vivo-derived cells are cultured. Use of the amniotic membrane also contributes the improvement of survival, so that the biological tissue sheet of the present invention can offer high treatment effect when the transplantation is carried out. In particular, in the biological tissue sheet produced by the method using amniotic membrane and collagen gel, a cell layer with extremely high density is formed and survival after transplantation can be extremely enhanced.

The biological tissue sheet of the present invention is used for regeneration (reconstruction) of skin epidermis, hair follicle epithelium, cornea epithelium, oral mucosal epithelium, intestinal tract mucosal epithelium, and respiratory tract mucosal epithelium. For example, the biological tissue sheet of the present invention can be directly transplanted to a tissue deficient site of the living body. Herein, "direct transplantation" means that the biological tissue sheet is transplanted without intervening other materials between a tissue deficient site and the biological tissue sheet. On the other hand, in order to allow the other materials to be intervened between them, the biological tissue sheet of the present invention (excluding the case where the second amniotic membrane is used in the production process) can be transplanted to a tissue deficient site of the living body. For example, the biological tissue sheet can be transplanted to the tissue deficient site via another amniotic membrane (second amniotic membrane) that is different from the amniotic membrane used as a culture substrate. Specifically, after the amniotic membrane (second amniotic membrane) is transplanted to the tissue deficient site, the biological tissue sheet that has been produced in advance can be transplanted to the amniotic membrane. With such a transplantation, since the amniotic membrane exists as a base material, cells contained in the biological tissue sheet are expected to be proliferated efficiently and well migrated to the surrounding. That is to say, a cell layer constituting the biological tissue sheet is expected to expand quickly, so that high treatment effect can be obtained. On the other hand, the tissue deficient site is covered with the amniotic membrane, and thereby protected from the outside, which contributes the improvement of the therapeutic effect.

The present invention further provides a new method of preparing skin epidermal cells based on the findings that proliferation and migration of epidermal cells on the amniotic membrane are excellent. The preparing method of the present invention includes the following steps: (A) seeding epidermal cells on amniotic membrane; (B) culturing and proliferating the epidermal cells; and (C) step of collecting the proliferated skin epidermal cells. In the preparing method of the present invention, cells are proliferated efficiently and the migration of cells to the surrounding becomes good. Therefore, a large area of cell layer (cultured epithelium) can be produced for a short time. Herein, collection and culturing of the skin epidermal cells can be a routine method (see mentioned above). Furthermore, for collection of the proliferated epidermal cells, physical means (peeling by using a cell scraper, and the like), enzymatic means (treatment with dispase or trypsin), and the like, can be used. Note here that epidermal cells may be collected in a state of the layer or may be collected in a state in which cells are divided into individual cells.

Hereinafter, the present invention is described specifically with reference to Examples, however, the present invention is not necessarily limited to the following Examples.

### [Example 1]

Production of three-dimensional cultured skin sheet (cultured epidermal sheet) using amniotic membrane

### 1. Preparation of amniotic membrane

### 1-1. Collection of amniotic membrane

After giving a pregnant woman who does not have a systemic complication and would undergo caesarean section sufficient informed consent together with an obstetrician in advance, amniotic membrane was obtained at the time of the caesarean section in the operation room. The operation was carried out cleanly. In accordance with the operation work, the operators washed hands, and then wore a special gown. Before delivery, a clean vat for obtaining amniotic membrane and physiologic saline for washing were prepared. After delivery, the placenta tissue was transferred to the vat and amniotic membrane tissue was manually detached from the placenta. A portion where amniotic membrane and placenta were strongly attached to each other was separated from each other with scissors.

### 1-2. Treatment of amniotic membrane

Treatment process of amniotic membrane included: (1) washing, (2) trimming, and (3) storing sequentially in this order. Throughout all the processes, operation is desired to be carried out in a clean draft. For all containers and instruments for use, sterilized ones were used, and for dishes, etc. sterilized disposable ones were used. The obtained amniotic membrane was washed for removing blood component attached thereto and further washed with a sufficient amount of physiological saline (0.005% ofloxacin was added). Then, the amniotic membrane was washed three times in total with a phosphate buffer solution (PBS) containing penicillin-streptomycin (50 IU). Then, the amniotic membrane was transferred into a dish and cut and divided into the size of about 4x3 cm with scissors. After the amniotic membranes were divided, amniotic membranes in good conditions were selected based on the shape, thickness, or the like.

### 1-3. Storage of amniotic membrane

One cc each of stock solution was placed in 2 cc sterilized cryotube and one sheet each of amniotic membrane, which had been obtained, washed and selected, was placed and labeled, and then stored in a deep freezer at -80°C. For the stock solution, 50% sterilized glycerol in DMEM (Dulbecco's Modified Eagle Medium: GIBCOBRL) was used. The expiration date for use of stored amniotic membrane was determined at 3 months and expired amniotic membrane was disposed of by incineration. Note here that instead of such storing treatment, the following treatment to the epithelium may be carried out.

### 1-4. Treatment of amniotic epithelium

Amniotic membrane stored at -80°C was thawed at room temperature, and then washed twice with a phosphate buffer solution (PBS) containing penicillin-streptomycin (50 IU). Amniotic membrane after washing was soaked in a 0.02% EDTA solution (Nacalai tesque) (in 100 mm culture dish) and reacted in a CO₂ incubator at 37°C for one hour. After reaction, the amniotic membrane was washed twice with a sufficient amount of PBS, and then the epithelium was manually denuded out by using a cell scraper (Nunc, USA). Note here that, it was confirmed that one layer of the amniotic epithelium was completely denuded by this procedure process by optical microscope and electron microscope (scanning electron microscope) operations (data are not shown).

### 2. Preparation of epidermal keratinocytes

### 2-1. Collection of skin

A small piece of skin is collected in accordance with skin biopsy. The site to be collected was preventively disinfected with povidone iodine and was subjected to external application of an antifungal agent in advance for about three days.

### 2-2. Serum free culture method of epidermal keratinocytes

Adipose tissue and dermis are removed as much as possible from the skin piece with scissors and washed with Dulbecco's phosphate buffer (PBS) several times. The skin is sterilized with 70% ethanol for one minute. The skin is washed with PBS, then cut into a strip shape with the size of about 3 mm ×10 mm, dipped in Dispase solution (Dispase II, Goudou Shusei, 250 units/ml, Dulbecco's Modified MEM medium; DMEM) and stood still overnight at 4°C. On the following day, by using forceps, epidermis is peeled off from dermis. The peeled dermis is subjected to fibroblasts culture. The peeled epidermis is washed with DMEM, then washed with PBS, and dipped into 0.25% trypsin solution to carry out treatment at 37°C for 10 minutes. The epidermis is transferred to a plastic petri dish containing a trypsin neutralization solution, is disentangled by using forceps, and transferred to 50 ml sterilization tube. PBS is added so as to prepare a suspending solution of epidermal keratinocytes. The number of cells is counted and the cells are subjected to centrifugation at 1000 rpm for 5 minutes, so that the cells are precipitated. Supernatant is sucked and the cells are suspended in a MCDB 153 medium that is a serum free medium, which is seeded at the rate of 2 to 3 × 10⁶ cells/10 ml culture solution for each 100 mm petri dish coated with collagen (ASAHI TECHNO GLASS CORPORATION, type I collagen coated dish; 4010-010). On the following day, the culture solution is exchanged, and later than that day, the culture solution is exchanged every other day. At the time when the cell density becomes about 70% to 80%, subculture is carried out.

### 3. Preparation of fibroblasts

After washing with DMEM, the peeled dermis is cut into strips with the size of 1 to 2 mm × 1 to 2 mm by using a surgical knife. The cut dermis strip is brought into close contact with a dish coated with type I collagen at intervals of about 1 cm. Then, the dermis is stood still in a CO₂ incubator for 30 minutes so as to be brought into close contact the dish completely. Thereafter, about 5 ml of DMEM medium containing 10% fetal bovine serum is added and stood still for seven days. On day 7, initial exchange of the culture solution is carried out. It is confirmed that fibroblasts are migrated from the dermis strip. At the stage when cells are proliferated and migrated to 5 mm vicinity of the dermis strip, subculture is carried out. The dermis is washed with PBS, and then a solution containing 0.125% trypsin and 0.05% EDTA is added and treated at 37°C for three minutes. After it is confirmed through a microscope that cells are detached from the bottom surface of the dish, 3 ml trypsin inhibitor is added and the cells are collected and transferred to 50 ml tube. By using PBS, remaining cells are collected and subjected to centrifugation at 1000 rpm for five minutes, so that cells are precipitated. The supernatant is sucked, and then a DMEM medium containing 10% fetal bovine serum is added so as to prepare a cell suspending solution, which is seeded on a cell culture dish. The cell density of subculture is about 1:3. The cells are cryopreserved appropriately. As a cryopreservation Solution, 10% glycerol, 20% FCS and 70% DMEM are used, and stored in liquid nitrogen.

### 4. Preparation of neutralized collagen gel

A neutralized collagen solution (final concentration of collagen: 1 mg/ml) is produced at 4°C by mixing one volume of 0.1N NaOH, one volume of 8 times concentration DMEM, ten volumes of 20% FCS/DMEM to six volumes of type I collagen solution (cell matrix type 1A: 3 mg/ml: Nitta Gelatin Inc.). One ml each of the neutralized collagen solution is dropped into 24 mm diameter culture insert (Corning-Costar) and stood still at room temperature for 10 minutes so as to be gelled. Fibroblasts in a logarithmic growth phase, which has been prepared in advance (cells are subjected to Dispase treatment to peel off epidermis and the remaining dermis is subcultured for 5-10 generations by an outgrowth method, and thus the subcultured cells are used) are adjusted to the concentration of 5×10⁵ cells/ml and 10% FCS/DMEM. This cell suspension (2 volumes) is mixed with a neutralized collagen solution (8 volumes) so as to prepare a neutralized collagen solution containing cells (final concentration of collagen: 0.8 mg/ml). To each culture insert, 3.5 ml each of this solution is added, and the culture insert is stood still in a CO₂ incubator (37°C, 5% CO₂). After 30 minutes, it is confirmed that the solution is gelled. Thereafter, 10% FCS/DMEM is added so that gel is dipped therein (3 ml is added to the inside of the culture insert, and 3 ml is added to the outside of the culture insert) and static culture is carried out for five days. On day 2 after culture is started, the gel starts to shrink. The proliferation of fibroblasts can be observed under phase contrast microscope.

### 5. Adhesion of amniotic membrane

On day 5 after culture is started, the bottom surface of the collagen gel is brought into close contact with membrane but the upper part of the collagen gel is shrunk to have a thickness of 2 to 3 mm. The preserved amniotic membrane (amniotic membrane from which epithelium has been removed) is washed with PBS twice and then washed with a culture solution for keratinocytes once. The amniotic membrane is transferred to a culture insert with the side of parenchymal cells facing downward and brought into close contact with collagen gel by using forceps. By using forceps, the collagen gel is expanded so that wrinkles are not generated and the periphery of the amniotic membrane is brought into close contact with the side wall of the culture insert, which is transferred to the inside of a CO₂ incubator and stood still at 37°C for 30 minutes.

### 6. Seeding of keratinocytes

The keratinocytes prepared in 2-2 are detached from the dish and collected by using trypsin - EDTA. The keratinocytes are subjected to centrifugation at 1000 rpm for five minutes to remove the supernatant. The cells are suspended to the concentration of 200 million cells /0.25 ml. The cell suspension (0.25 ml) is plated to the amniotic membrane placed inside the culture insert, transferred to a CO₂ incubator and stood still in the incubator for 1.5 to 2.0 hours so that keratinocytes are brought into close contact with the amniotic membrane. Thereafter, I ml of medium for proliferating epidermal cells is gently added to the inside of the culture insert and further I ml of the medium is added to the outside of the culture insert. On the following day, a medium for proliferating epidermal cells is gently added to the inside of the culture insert and 1 ml of the medium for proliferating epidermal cells is added also to the outside of the culture insert.

### 7. Culture under vapor phase conditions

On day 3 following the plating of epidermal cells onto amniotic membrane, air exposure (air lifting) is carried out. Sterilized filter paper is set to a maintaining vessel for air exposure, a stratifying medium is added so that the filter paper is dipped (about 9 ml). The culture solution inside the culture insert is carefully removed and the culture insert is transferred onto the filter paper and cultured in a CO₂ incubator. The culture solution is exchanged every other day. By air exposure for 7 to 14 days, a three-dimensional cultured skin is completed. The stratifying medium is prepared as follows. Dulbecco's Modified MEM medium: F-12 medium = 1:1, calcium concentration; 1.95 mM, monoethanolamine; 0.1 mM, 0-phosphoethanolamine; 0.1 mM, insulin; 5 ug/ml, hydrocortisone; 0.4 ug/ml, L-glutamine; 4mM, Adenin; 0.18 mM, transfferin; 5 ug/ml, selenious acid; 53 nM, triiodothyronine; 20pM, serine; 1 mM, choline chloride; 0.64 mM, linoleic acid; 2 ug/ml, FCS; 2%.

The cultured epidermal sheet obtained by the above-mentioned operation can be easily detached from the bottom surface of the dish or a collagen matrix. Since the sheet produced by a conventional technique may shrink, it is necessary to use a chitin film (BESCHITIN W) as a support. Furthermore, the conventional sheet is often broken. However, according to the above-mentioned method, a strong sheet is prepared and shrinkage of the sheet is not observed, making it not necessary to use the supporting materials.

### 8. Histological analysis

When a cultured epidermal sheet is produced by the above-mentioned method, on day 7 following the air exposure, epidermis had 5 to 8 layers and the formation of horny cell layer was observed. The cultured epidermal sheet had substantially the same structure as the normal human skin. The histological findings of the stratified keratinocytes shows a cell construct including one layer of basal cell-like cells and 5 to 8 layers of cells stratified and differentiated on the basal cell-like cells (Fig. 1). When a cultured epidermal sheet is produced by using cells, which have been subcultured for three generations, at about fourth week flowing the collection of the skin, the cultured epidermal sheet can be used. The cultured area is increased to several thousand times according to calculation.
On the other hand, a specimen is prepared over time before and after the air exposure, and HE staining is carried out. By using a specimen produced nine days after the air exposure is carried out, a frozen tissue piece is prepared and subjected to immunohistochemical staining by using a Histofine SAB - AP kit (NICHIREI CORPORATION). As a substrate. New fuchsia is used. Antigens to be used include: monoclonal antibody; laminin 5 (GB3, Sera Lab), type IV collagen (MAB1910, Chemicon), type VII collagen (LH 7.2, YLEM), β4 integrin (MAB 1964, Chemicon), β1 integrin (P4CIO, Gibco BRL), desmoglein 1 (DG 3.10, Progen), plakoglobin (PG 5.1, Progen), desmoplakin (DP- 2.15, Progen), E cadherin (HECD -1, Takara), Pan -keratin (Dako), EGF receptor (Ab3, Oncogene Science), polyclonal antibody; Impolclin (Biomedical Technologies Inc.), and desmoglein 3 (Serotec). For confirmation of pemphigoid antigen, serum from a patient with pemphigoid and FITC labeled anti-human IgG antibody are used and subjected to observation under fluorescence microscope. In electron microscopy analysis, a specimen produced nine days after the air exposure was fixed and embedded by a routine method and observed through transmission electron microscopy (Hitachi 1td.).
According to HE findings before air exposure, 1 to 2 layers of keratinocytes exist and the formation of horny cell layers is not observed. On day 4 following the air exposure, stratification of keratinocytes is observed and the formation of 3 to 4 layers of spinous cell layers and clear horny cell layers is observed. On day 7, about 5 to 8 layers of spinous cell layers are observed and this state is reliably maintained until about 14th day. The sheet that is clearly stronger as compared with a conventional cultured epithelium sheet is formed. As a result, it was thought that operation was easily carried out and the sheet was easily handled even if a support material was not used. Immunohistologically, laminin 5, type IV collagen, type VII collagen, β4 integrin are expressed mainly in the epidermal basal cells of the basal layer, which showed different expression form from that of vivo epidermis. E cadherin, desmoglein 1 and desmoglein 3, which are intercellular adhesion molecules, and desmoplakin and plakoglobin, which are lined protein are expressed in the range from the basal layer to the spinous cell layer. An EGF receptor and β1 integrin are expressed in the range from the basal layer to a para-basal layer. Pemphigoid antigens are expressed linearly in a basal layer portion. According to the immunohistological findings, intercellular adhesion molecule and marker for differentiation are expressed approximately similar to those of vivo epidermis. However, it was thought that the expression of the component of the basal layer was insufficient. According to the findings of electron microscopy, desmosomes are well formed. In the basal layer portion, the formation of hemidesmosome was approximately favorable. However, the formation of the basal plate was partially observed but it was observed discontinuously. The formation of anchoring fibers was observed discontinuously.

### 9. Preservation of sheet

Produced cultured epidermal sheet can be frozen by using a small amount of stock solution with or without a carrier. Specifically, firstly, the sheet is cryopreserved in a -80°C freezer and on the following day, it is preserved in ultra-cold -150°C freezer. By preservation at -150°C, the shape of the sheet can be maintained for a long term. Actually, sufficient treatment effect can be obtained. Besides, the sheet can be preserved at 4°C by using a stock solution used for storing biomedical tissue. In this case, it is desirable that antioxidant be added.

### 10. Transplantation of cultured epidermal sheet

The detached sheet is transplanted in a way in which the surface that was attached to the plate covers a wounded surface, and pressing and fixation are carried out. Usually, the pressing and fixation can be sufficiently carried out by fixation with tape and pressing by bandage. Similar to the usual skin transplantation, the transplantation of a cultured sheet is also classified into autotransplantation using patient's own cells and heterotransplantation using other person's cells. The autotransplantation is excellent in survival but it takes a long time to produce a cultured sheet. Meanwhile, the heterotransplantation does not offer survival, but has an effect of biological dressing and survival is apparently obtained. Since the preservation of sheets has come to be possible, it is advantage that large amount of hetero cultured sheets are produced at one time and they can be used in case of necessity. For these reasons, hetero cultured sheets are mainly applied to patients with acute stage burn. Clinical application of hetero cultured sheets includes covering sites of burn, ulcus cruris, epidermolysis bullosa hereditaria, giant hairy nevus, scarring, a site from which split-layer skin has been harvested, and the like.

### [Example 2]

Production of three-dimensional cultured skin sheet (cultured epidermal sheet) (in the case where collagen gel is not used)

### 1. Collection of amniotic membrane and epidermal keratinocyte

Amniotic membrane and epidermal keratinocytes were prepared by the same procedure as described in Example 1.

### 2. Seeding of keratinocytes

Preserved amniotic membrane is washed with PBS twice and further washed with a culture solution for keratinocytes once. The amniotic membrane is attached to the bottom surface of a culture insert with the side of substantial cells facing downward. The keratinocytes that have been prepared in advance are detached from the dish and collected by using trypsin - EDTA. The keratinocytes are subjected to centrifugation at 1000 rpm for five minutes to remove the supernatant. The cells are suspended so that the concentration becomes 200 million cells /0.25 ml. The cell suspension (0.25 ml) is seeded to the amniotic membrane inside the culture insert, transferred to a CO₂ incubator and stood still in the incubator for 1.5 to 2.0 hours so that keratinocytes are brought into close contact with the amniotic membrane. Thereafter, I ml of medium for proliferating epidermal cells is gently added to the inside of the culture insert and further I ml of medium is added to the outside of the culture insert. On the following day, a medium for proliferating epidermal cells is gently added to the inside of the culture insert and 1 ml of medium for proliferating epidermal cells is added also to the outside of the culture insert.

### 3. Culture under vapor phase conditions

On day 3 following the plating of epidermal cells onto amniotic membrane, air exposure (air lifting) is carried out. Sterilized filter paper is set to a maintaining vessel for air exposure, a stratifying medium is added so that the filter paper is dipped (about 9 ml). The culture solution inside the culture insert is carefully removed and the culture insert is transferred onto the filter paper and cultured in a CO₂ incubator. The culture solution is exchanged every other day. By air exposure for 7 to 14 days, a three-dimensional cultured skin is completed. The stratifying medium is adjusted as follows. Dulbecco's Modified MEM medium: F-12 medium = 1:1, calcium concentration; 1.95 mM, monoethanolamine; 0.1 mM, 0-phosphoethanolamine; 0.1 mM, insulin; 5 ug/ml, hydrocortisone; 0.4 ug/ml, L-glutamine; 4mM, Adenin; 0.18 mM, transfferin; 5 ug/ml, selenious acid; 53 nM, triiodothyronine; 20pM, serine; 1 mM, choline chloride; 0.64 mM, linoleic acid; 2 ug/ml, FCS; 2%.

### 4. Histological analysis

When a cultured epidermal sheet was produced by the above-mentioned method, on day 7 following the air exposure, epidermis had 5 to 8 layers and the formation of horny cell layer was observed. The cultured epidermal sheet had substantially the same structure as the normal human skin (Fig. 2).

### [Example 3]

Production of three-dimensional cultured corneal epithelial sheet using amniotic membrane

### 1. Preparation of amniotic membrane

Amniotic membrane was prepared by the same procedure as described in Example 1.

### 2. Preparation of corneal epithelial cell

### 2-1. Procurement of cornea

Donor corneas were purchased from Northwest Lions Eye Bank (Seattle, USA).

### 2-2. Serum free culture method of corneal epithelial cells

Cornea is transferred to a petri dish containing Dulbecco's phosphate buffer (PBS) and the limbus is cut into strip with the size of 2 to 3 mm × 2 to 3 mm by using a surgical knife under stereoscopic microscope. The limbus strip is washed with PBS several times and was sterilized by dipping it into 70% ethanol for one minute. The strip is washed with PBS, dipped in Dispase solution (Dispase II, Goudou Shusei, 250 units/ml. Dulbecco's Modified MEM medium; DMEM) and stood still overnight (18 to 24 hours) at 4°C. On the following day, by using forceps, epithelium is peeled off from the substance under stereoscopic microscope. The peeled corneal epithelium is washed with DMEM, then washed with PBS, and dipped into 0.25% trypsin solution to carry out treatment at 37°C for 10 minutes. Epidermis is transferred to a plastic petri dish containing a trypsin neutralization solution, disentangled by using forceps, and transferred to 15 ml sterilization tube. PBS is added to prepare a corneal epithelial cell suspending solution. The number of cells is counted and the cells are subjected to centrifugation at 1000 rpm for 5 minutes, so that the cells are precipitated. Supernatant is sucked and the cells are suspended in an EpiLife medium that is a serum free medium, which is seeded at the rate of 1 to 2 × 10⁶ cells / 5 ml culture solution for each 60 mm petri dish coated with collagen (ASAHI TECHNO GLASS CORPORATION, type I collagen coated dish; 4010-020). On the following day, the culture solution is exchanged, and later than that day, the culture solution is exchanged every other day. At the time when the cell density becomes about 70% to 80%, subculture is carried out.
Note here that in the above-mentioned method, by using a serum free medium, corneal epithelial cells are cultured. However, a medium containing serum can be used as in the following procedures.
(1) Peel and remove endothelium cells from the corneal limbus tissue and excise conjunctiva.
(2) Immerse in dispase solution (Dispase I, Goudou Shusei, 250 units/ml, Dulbecco's Modified MEM medium; DMEM) and stand it still overnight (for 18 to 24 hours) at 4°C.
(3) Immerse in a 0.25% trypsin solution and treat at 37°C for 10 minutes.
(4) Peel only epithelium in a trypsin solution under microscope.
(5) Carry out pipetting and add the same amount of 30% FCS/DMEM so as to obtain suspension.
(6) Collect the remaining cells by PBS (-) and carry out centrifugation.
(7) Use a proper amount of culture solution so as to obtain a single cell suspension.

An example of cryopreservation conditions (including the composition of a stock solution) and melting conditions of the prepared corneal epithelial cells is shown bellow.
Cryopreservation conditions: lower the temperature to -20°C at the rate of 1°C/hour and then preserve in a nitrogen tank.
Composition of stock solution: 20% FCS / 10% DMSO / DMEM
Melting conditions: melt at 37°C as quickly as possible and dilute 10 times with PBS.

### 3. Preparation of fibroblasts

After washing with DMEM, the peeled dermis is cut into strips with the size of 1 to 2 mm × 1 to 2 mm by using a surgical knife. The cut dermis strip is brought into close contact with a dish coated with type I collagen at intervals of about 1 cm. Then, the dermis is stood still in a CO₂ incubator for 30 minutes so as to be brought into close contact the dish completely. Thereafter, about 5 ml of DMEM medium containing 10% fetal bovine serum is added and stood still for seven days. On day 7, initial exchange of the culture solution is carried out. It is confirmed that fibroblasts are migrated from the dermis strip. At the stage when cells are proliferated and migrated to 5 mm vicinity of the dermis strip, subculture is carried out. The dermis is washed with PBS, and then a solution containing 0.125% trypsin and 0.05% EDTA is added and treated at 37°C for three minutes. After it is confirmed through a microscope that cells are detached from the bottom surface of the dish, 3 ml trypsin inhibitor is added and the cells are collected and transferred to 50 ml tube. By using PBS, remaining cells are collected and subjected to centrifugation at 1000 rpm for five minutes, so that cells are precipitated. The supernatant is sucked, and then a DMEM medium containing 10% fetal bovine serum is added so as to prepare a cell suspending solution, which is seeded on a cell culture dish. The cell density of subculture is about 1 : 3. The cells are cryopreserved appropriately. As a cryopreservation solution, 10% glycerol, 20% FCS and 70% DMEM are used, and stored in liquid nitrogen.

### 4. Preparation of neutralized collagen gel

A neutralized collagen solution (final concentration of collagen: 1 mg/ml) is produced at 4°C by mixinig one volume of 0.1N NaOH, one volume of 8 times concentration DMEM, 10 volumes of 20% FCS/DMEM to six volumes of type 1 collagen solution (cell matrix type 1A: 3 mg/ml: Nitta Gelatin Inc.). One ml each of the neutralized collagen solution is dropped into 24 mm diameter culture insert (Corning-Costar) and stood still at room temperature for 10 minutes so as to be gelled. Fibroblasts in a logarithmic growth phase, which has been prepared in advance (cells are subjected to dispase treatment to peel off epidermis and the remaining dermis is subcultured for 5-10 generations by an outgrowth method, and thus the subcultured cells are used) are adjusted to the concentration of 5×10⁵ cells/ml and 10% FCS/DMEM. This cell suspension (2 volumes) is mixed with a neutralized collagen solution (8 volumes) so as to prepare a neutralized collagen solution containing cells (final concentration of collagen: 0.8 mg/ml). To each culture insert, 3.5 ml each of this solution is added, and the culture insert is stood still in a CO₂ incubator (37°C, 5% CO₂). After 30 minutes, it is confirmed that the solution is gelled. Thereafter, 10% FCS/DMEM is added so that gel is dipped therein (3 ml is added to the inside of the Culture insert, and 3 ml is added to the outside of the culture insert) and static culture is carried out for five days. On day 2 after culture is started, the gel starts to shrink. The proliferation of fibroblasts can be observed under phase contrast microscope.

### 5. Adhesion of amniotic membrane

On day 5 after culture is started, the bottom surface of the collagen gel is brought into close contact with membrane but the upper part of the collagen gel is shrunk to have a thickness of 2 to 3 mm. The preserved amniotic membrane is washed with PBS twice and then washed with a culture solution for keratinocytes once. The amniotic membrane is transferred to a culture insert with the side of parenchymal cells facing downward and brought into close contact with collagen gel by using forceps. By using forceps, the collagen gel is expanded so that wrinkles are not generated and the periphery of the amniotic membrane is brought into close contact with the side wall of the culture insert, which is transferred to the inside of a CO₂ incubator and stood still at 37°C for 30 minutes.

### 6. Plating of corneal epithelial cells

The corneal epithelial cells prepared in 2-2 are detached from the dish and collected by using trypsin - EDTA. The corneal epithelial cells are subjected to centrifugation at 1000 rpm for five minutes to remove the supernatant. The cells are suspended to the concentration of 200 million cells /0.25 ml. The cell suspension (0.25 ml) is seeded on the amniotic membrane inside the culture insert and transferred to a CO₂ incubator and stood still in the incubator for 1.5 to 2.0 hours so that keratinocytes are brought into close contact with the amniotic membrane. Thereafter, medium for proliferating epidermal cells is gently added (3 ml to the inside of the culture insert and 3 ml to the outside of the culture insert). Culture is continued for further 14 days in the liquid phase. On day 3 following the sowing of corneal epithelial cells, the culture solution is exchanged with a culture solution for stratification (see below), and later than that day, culture solution is exchanged every other day.
The medium for stratification is prepared as follows. Dulbecco's Modified MEM medium: F-12 medium = 1 : 1, calcium concentration; 1.95 mM, monoethanolamine; 0.1mM, 0-phosphoethanolamine; 0.1mM, insulin; 5 ug/ml, hydrocortisone; 0.4 ug/ml, L-glutamine; 4mM, Adenin; 0.18 mM, transfferin; 5 ug/ml, selenious acid; 53 nM, triiodothyronine; 20 pM, serine; 1 mM, choline chloride; 0.64 mM, linoleic acid; 2 ug/ml, and FCS; 2%.

### 7. Culture under vapor phase conditions

On day 14 following the sowing of corneal epithelial cells, air exposure (air lifting) is carried out. Sterilized filter paper is set to a maintaining vessel for air exposure, a stratifying medium is added so that the filter paper is dipped (about 9 ml). The culture solution inside the culture insert is carefully removed and the culture insert is transferred onto the filter paper and cultured in a CO₂ incubator. On day 3, the culture solution is exchanged. By air exposure for 3 days, a cultured cornea is completed.

### 8. Histological analysis

On day 3 following the air exposure, corneal epithelium has 3 to 4 layers and the formation of horny cell layer is observed. The cultured corneal epithelial sheet has substantially the same structure as the normal human cornea.

### [Example 4]

Evaluation of characteristics of amniotic membrane as cell culture substrate

### 1. Proliferation and migration test 1 of epidermal keratinocytes on amniotic membrane

Amniotic membrane from which epithelium has been removed is placed and brought into contact with collagen gel containing fibroblasts in a way in which the side on which the epithelium existed facing upward. Next, on the amniotic membrane, a doughnut-shaped ring made of stainless steel (inner diameter: 6 mm, height: 2 mm) is placed, epithelium keratinocyte suspension is plated inside (6 mm-opening portion). Note here that both amniotic membrane and epithelium keratinocyte suspension are prepared by the method described in Example 1.
Two days after, the ring was removed and stratification by air exposure was started. On day 1 and day 10 following the stratification, migration of epidermal keratinocytes toward the surrounding was observed. Comparative subjects (control group) were made by the same conditions except that amniotic membrane was not used.
Fig. 3 shows a state inside a petri dish on day 1 and day 10 following the stratification. Right pictures of Fig. 3 show the results (upper picture: day 1, lower picture: day 10) of test group (the case where epidermal keratinocytes are cultured on the amniotic membrane with which collagen gel containing fibroblasts is brought into contact). Left pictures of Fig. 3 show the results of control group (the case where epidermal keratinocytes are cultured on collagen gel containing fibroblasts). Circles or spots observed in the middle region of the petri dish are cells (cell layer).
In the test group (left), from the first day to tenth day, cell layer is enlarged significantly. That is to say, it is shown that cells are well proliferated and migration toward the surrounding is proceeded. On the other hand, in the control group, on day 1 and day 10, significant change in the size of the cell layer is not recognized. From the above-mentioned results, it is clear that the proliferation rate of cells is high and the migration is significantly proceeded on amniotic membrane.

### 2. Proliferation and migration test 2 of epidermal keratinocytes on amniotic membrane (combination with three-dimensional culture method)

Firstly, three-dimensional culture was carried out by the procedures described in Example 1 (culturing of epidermal keratinocytes on the amniotic membrane attached to collagen gel followed by carrying out air exposure), so that a cultured epidermal sheet on which a cell layer was formed on amniotic membrane (sheet on day 7 after stratification by air exposure is started) was formed. Meanwhile, amniotic membrane from which epithelium had been removed was placed in a state in which it was expanded and brought into contact with collagen gel containing fibroblasts in a way in which the side on which the epithelium existed facing upward. Next, the cultured epidermal sheet was punched in a shape of circle with diameter of about 8 mm and placed on the amniotic membrane on collagen gel containing fibroblasts and stood still. Then, expansion of the epidermal keratinocyte layer was observed on day 1, day 7, day 10 and day 14. A sheet to be compared was prepared by producing the same conditions except that amniotic membrane was not used (control group 1), and a sheet to be compared was also prepared by directly placing a cell layer obtained by three-dimensional culture without using amniotic membrane (cell layer obtained by directly sowing epidermal keratinocytes onto collagen gel and then culturing thereof) on collagen gel containing fibroblasts (control group 2). Note here that amniotic membrane and epidermal keratinocyte suspension were prepared by the method described in Example 1.
Fig. 4 shows a state in a petri dish on day 1, 7, 10 and 14 after culturing is started. Left pictures of Fig. 4 show the results (pictures of day 1, day 7, day 10 and day 14 are shown from the top in this order) of test group (the case where epidermal keratinocytes are cultured on the amniotic membrane with which collagen gel containing fibroblasts is brought into contact). Middle pictures show the results of control group 1 (the case where cultured epidermal sheet is placed and cultured on collagen gel containing fibroblasts). Similarly, right pictures show the results of control group 2 (the case where a cell layer obtained by three-dimensional culturing without using amniotic membrane).
In Fig. 4, cells (cell layer) are shown substantially in the middle of the petri dish. In test group (left), the cell layer is expanded significantly over time. That is to say, it is shown that cells are well proliferated and the migration of cells toward the surrounding is proceeded. Meanwhile, in control I (middle), the cell layer is recognized to be slightly expanded over time, but the degree is significantly different from the case of the test group. Furthermore, in control group 2 (right), no significant change is observed during the observation term. As mentioned above, it is clear that the cells constituting the cultured epidermal sheet obtained by three-dimensional culture are also proliferated at a high proliferation rate and that migration of cells toward the surrounding is proceeded significantly.

### 3. Summary

From the results 1 and 2, it was determined that on amniotic membrane, epidermal keratinocytes were well proliferated and the migration ability of cells were well exhibited. Furthermore, according to the result of 2, it was thought that a method of collecting epidermal keratinocytes cultured on the amniotic membrane placed on a collagen gel containing fibroblasts together with amniotic membrane and transplanting them on another amniotic membrane which has been transplanted on the skin defective portion in advance is an excellent epidermal reconstruction method. The transplantation operation is shown below.
(1) Defective injury of full thickness and a part including subcutaneous tissue, firstly, debridement is carried out so as to remove necrotic tissue. Specifically, 1% xylocaine E is injected to the vicinity of the defective injury, followed by removing necrotic tissue with surgical knife or scissors so as to flatten the bottom surface of the ulcer. Next, artificial dermis is transplanted and tie over fixation is carried out.
(2) One to two weeks later, the tie over fixation is removed and survival of artificial dermis is confirmed. Specifically, it is visually confirmed that artificial dermis is brought into close contact with the bottom surface of the ulcer. Then, the artificial dermis is moved side to side by fingers and it is confirmed that the artificial dermis does not move. It is visually confirmed that exudates or blood is retained beneath the artificial dermis. Subsequently, on the survived artificial dermis, amniotic membrane (amniotic membrane from which epithelium component has been removed and which is confirmed that bacterial typing is negative is cryopreserved is heated to 37°C and washed with sterilized physiological saline) is transferred and fixed by bandage, tape, tie-over, and the like.
(3) After the fixed state is maintained for several days, it is confirmed that amniotic membrane survives on the artificial dermis. Specifically, it is visually confirmed that exudates or blood is retained between amniotic membrane and artificial dermis. Furthermore, the artificial dermis is moved side to side by fingers and it is confirmed that the artificial dermis does not move.
(4) Three-dimensional cultured epidermal sheet (sheet including amniotic membrane and cell layer or sheet including only a cell layer) is transplanted by patch graft (for example, one patch has a diameter of 15 mm). The sheet is fixed for three days and after that day, disinfection treatment (0.05% Hibitane and Isodine for disinfection) is carried out every two days.
(5) It is thought that cells for constructing a three-dimensional cultured epithelium sheet are well proliferated and migrated when they are transplanted on amniotic membrane. As a result, a cell layer is quickly expanded and high treatment effect is expected to be exhibited.

### INDUSTRIAL APPLICABILITY

The application of biological tissue sheet provided by the present invention is wide. The biological tissue sheet can be used for regenerating (reconstructing) skin epidermis, corneal epithelium, oral mucosa epithelial cells, respiratory tract mucosa epithelial cells, intestinal tract mucosa epithelial cells, and the like. Among them, it can be suitably used for regeneration of skin epidermis or corneal epithelium.
Furthermore, the biological tissue sheet of the present invention can be used for gene therapy. The gene therapy is largely classified into in vivo method and ex vivo method. In the in vivo method, gene is directly introduced into the living body and in the ex vivo method, once a cell is taken out, gene is introduced the cell and the cell is returns to the body again.
In the view of the current state of the art of gene therapy, only by introducing a gene into cultured skin, cultured corneal epithelium, and cultured intestinal tract mucosa epithelial cell, the ex vivo method can be carried out. The effectiveness of gene introduction to keratinocytes by using various virus vectors has been shown. In particular, when an adenovirus vector is used, gene can be introduced into substantially 100% of keratinocytes. In the gene therapy in the field of dermatology, in the treatment of genetic disease such as epidermolysis bullosa hereditaria caused by abnormality of type VII collagen and laminin 5, and the like, it is expected that effective treatment can be carried out by introducing normal gene into an autologous cultured epithelium sheet. Furthermore, in systemic diseases such as diabetes and hemophilia, which are caused by deficiency of molecules in the body, cultured skin may be used as so-called delivery system of producing and supplementing deficient molecules by introducing a gene into keratinocyte. It is expected that the application of cultured body tissues is increased in the future.

Note here that in many countries, xeno- and allo-geneic transplantation has problems in terms of ethical aspect and safety confirmation. Under present circumstances, autotransplantation has been exclusively promoted. In this respect, it is thought that autotransplantation of a three-dimensional cultured biological tissue is promoted in the future.

The present invention is not limited to the description of the above embodiments and Examples. A variety of modifications, which are within the scopes of the claims and which can be easily achieved by a person skilled in the art, are included in the present invention.
All of the articles, publication of unexamined patent application, and Patent Gazette cited herein are incorporated in their entirety by reference.

## Claims

1. A biological tissue sheet comprising in vivo-derived cells proliferated on amniotic membrane in the absence of a xenogeneic animal cell.

2. The biological tissue sheet according to claim 1, wherein the in vivo-derived cells are proliferated in a state in which the amniotic membrane is placed on a collagen gel containing human fibroblasts.

3. The biological tissue sheet according to claim 1 or 2, wherein the in vivo-derived cells are proliferated by using a serum free medium.

4. The biological tissue sheet according to claim 1 or 2, wherein the in vivo-derived cells are proliferated by using a medium including only serum derived from a recipient as a serum component.

5. The biological tissue sheet according to any of claims 1 to 4, wherein the in vivo-derived cells are cells derived from corneal epithelium, conjunctival epithelium, skin epidermis, hair follicle epithelium, oral mucosa, respiratory tract mucosa or intestinal tract mucosa.

6. The biological tissue sheet according to any of claims 1 to 5, wherein the amniotic membrane is amniotic membrane from which epithelium has been removed.

7. The biological tissue sheet according to any of claims to 6, comprising the amniotic membrane as a culture substrate in addition to proliferated cells.

8. The biological tissue sheet according to claim 7, which is a transplantation material to be transplanted to a defective tissue site via second amniotic membrane.

9. The biological tissue sheet according to any of claims 1 to 6, comprising cells obtained by placing in vivo-derived cells, which are proliferated on amniotic membrane placed on a collagen gel containing human fibroblasts, on second amniotic membrane together with the amniotic membrane in the absence of a xenogeneic animal cell, and further proliferating thereof.

10. A forming method of a biological tissue sheet, the method comprising the following steps:
(a) preparing in vivo-derived cells;
(b) sowing the in vivo-derived cells on amniotic membrane; and
(c) culturing and proliferating the in vivo-derived cells in the absence of a xenogeneic animal cell.

11. The forming method according to claim 10, wherein the step (b) consists of the following steps:
(b-1) culturing human fibroblasts in a collagen gel; and
(b-2) placing amniotic membrane on the collagen gel, followed by plating the in vivo-derived cells on the amniotic membrane.

12. The forming method according to claim 10 or 11, further comprising the following step:
(d) after the in vivo-derived cells are proliferated, bringing the outermost surface layer into contact with air.

13. The forming method according to claim 11 or 12, further comprising the following steps:
(e) collecting the in vivo-derived cells together with the amniotic membrane; and
(f) placing the collected in vivo-derived cells and the amniotic membrane on second amniotic membrane with a side of the amniotic membrane facing downward, followed by culturing and proliferating the in vivo-derived cells.

14. The forming method according to any of claims 10 to 13, wherein the step (c) is carried out by using a serum free medium.

15. The forming method according to any of claims 10 to 13, wherein the step (c) is carried out by using a medium including only serum derived from a recipient as a serum component.

16. The forming method according to any of claims 10 to 15, wherein the in vivo-derived cells are cells derived from corneal epithelium, conjunctival epithelium, skin epidermis, hair follicle epithelium, oral mucosa, respiratory tract mucosa or intestinal tract mucosa.

17. The forming method according to any of claims 10 to 16, wherein the amniotic membrane is amniotic membrane from which epithelium has been removed.

18. A method of preparing a skin epidermal cell, the method comprising the following steps:
(A) plating skin epidermal cells onto amniotic membrane;
(B) culturing and proliferating the skin epidermal cells; and
(C) collecting the proliferated skin epidermal cells.

19. A transplantation method using the biological tissue sheet according to any of claims 1 to 9 as a transplantation material.
